(19) Europäisches Patentamt
European Patent Office
Office européen des brevets

(11) Publication number: **0 430 579 A2**

(12) **EUROPEAN PATENT APPLICATION**

(21) Application number: **90312709.0**

(22) Date of filing: **22.11.90**

(51) Int. Cl.[5]: **C07C 69/63**, C07C 67/347, C07F 9/10, G11B 5/84

(30) Priority: **22.11.89 US 440871**

(43) Date of publication of application: **05.06.91 Bulletin 91/23**

(84) Designated Contracting States: **DE ES FR GB IT Bulletin**

(71) Applicant: **MINNESOTA MINING AND MANUFACTURING COMPANY**
**3M Center, P.O. Box 33427**
**St. Paul, Minnesota 55133-3427(US)**

(72) Inventor: **Behr, Frederick E., c/o Minnesota Mining and Manufact. Co., 2501 Hudson Road, P.O. Box 33427**
**St. Paul, Minnesota 55133-3427(US)**
Inventor: **Hagen, Donald F., c/o Minnesota Mining and Manufact. Co., 2501 Hudson Road, P.O. Box 33427**
**St. Paul, Minnesota 55133-3427(US)**
Inventor: **Johnson, James D., c/o Minnesota Mining and Manufact. Co., 2501 Hudson Road, P.O. Box 33427**
**St. Paul, Minnesota 55133-3427(US)**

(74) Representative: **Baillie, Iain Cameron et al**
**c/o Ladas & Parry Isartorplatz 5**
**W-8000 München 2(DE)**

(54) **Fluoroaliphatic-group-substituted esters and method of making the same.**

(57) Fluorine-containing compounds comprising fluoroaliphatic-group-substituted esters derived from
(A) ethylenically unsaturated ester comprising a residue of an aliphatic polyol compound substituted with at least one acyl group derived from an ethylenically unsaturated fatty acid, said acyl group having at least one non-terminal carbon-carbon double bond, and
(B) fluoroaliphaticsulfonyl chloride or bromide compound.

EP 0 430 579 A2

# FLUOROALIPHATIC-GROUP-SUBSTITUTED ESTERS AND METHOD OF MAKING THE SAME

This invention relates to certain perfluoroaliphatic-group-substituted organic compounds prepared by addition of fluoroaliphaticsulfonyl chloride or bromide or perfluoroaliphatic iodide compounds to certain terminally unsaturated compounds such as terminally unsaturated aliphatic acids, esters and olefins and certain non-terminally unsaturated compounds. In another aspect this invention relates to 1-palmitoyl-2-(8,8-difluoro)palmitoyl-sn-glycero-3-phosphorylcholine and difluoromethylene-containing fatty acids.

U.S. Pat. No. 2,951,051 (Tiers) describes perfluoroaliphatic-group-substituted acids which can be prepared either by desulfonylative addition of perfluorinated sulfonyl chloride or addition of perfluoroaliphatic iodide to a terminally unsaturated aliphatic carboxylic acids. The '051 patent further states that "simple" derivatives of these acids can be formed by formal dehydration reactions to form acyl halide, anhydride, amide, amidine, nitrile, hydrazide, peroxide, ester and orthoester derivatives.

U.S. Patent No. 3,818,074 (Ahlbrecht) describes certain terminally unsaturated aliphatic acid esters of omega-perfluoroalkyl substituted alkanols. The '074 patent specifically describes reaction of perfluoroaliphaticsulfonyl chloride, bromide or perfluoroaliphatic iodide compounds with certain terminally unsaturated aliphatic alcohols, acids and esters (for example, see col. 2).

U.S. Pat. No. 2,965,659 (Tiers) describes the reaction of fluoroaliphaticsulfonyl chlorides or bromides with olefins which are capable of copolymerization with another olefin under free-radical initiation, and which contain at least one hydrogen atom attached to an olefinic carbon atom, the remaining three valences of the olefinic carbon atoms being satisfied by radicals having Hammett sigma (para) parameter values whose algebraic sum is not greater than about 0.4. The '659 patent describes certain olefins having non-terminal carbon-carbon double or triple bonds which are reactive with fluorocarbon sulfonyl chlorides or bromides (for example, Examples 11, 12 and 13 and col. 5, lines 50-66) but it does not describe any organic acids, esters, or anhydrides having non-terminal carbon-carbon double bonds which are not either cyclic compounds or alpha-beta-unsaturated carbonyl compounds.

Certain fluorine-containing fatty acids and fatty acid esters have been described in Gent et al., J. Amer. Chem. Soc., 98, 3749 (1976) and Gent et al., Proc. Nat'l. Acad. Sci., 75, 630 (1978). The first Gent et al. reference describes the synthesis of 1-palmitoyl-2-(8,8-difluoro)palmitoyl-sn- glycero-3-phosphorylcholine using the keto acid and $MoF_6$ to convert a ketone group into a gem-difluoromethylene group. The second Gent et al. reference describes difluoromethylene-containing fatty acids synthesized from the corresponding keto acid methyl esters by a technique involving $MoF_6$. Neither of these references describes fluoroaliphatic-group-substituted compounds or Applicants' methods of preparing such compounds.

The present invention provides fluorine-containing compounds comprising fluoroaliphatic-group-substituted esters derived from

(A) ethylenically unsaturated ester (hereinafter, for brevity, sometimes referred to as unsaturated ester) comprising a residue of an aliphatic polyol compound substituted with at least one acyl group (i.e., RCO-) derived from an ethylenically unsaturated fatty acid, said acyl group having at least one, and preferably two or more, non-terminal carbon-carbon double bonds, and

(B) fluoroaliphaticsulfonyl halide (i.e., bromide and/or chloride) compound.

The novel fluoroaliphatic-group-substituted esters of this invention can be useful as emulsifiers, surfactants, lubricants or anti-static agents. Said compounds are particularly useful as emulsifiers or surfactants in compositions comprising emulsions of highly fluorinated liquids, e.g perfluorotributylamine, and non-fluorinated, immiscible liquids.

The invention also provides a method of preparing said fluoroaliphatic-group-substituted esters. The fluoroaliphatic-group-substituted esters can be directly prepared from the unsaturated ester and fluoroaliphaticsulfonyl halide compound. This method can provide a convenient means of directly substituting many naturally occurring unsaturated esters such as lecithin and animal or vegetable oils or fats and many other commercially available, non-naturally occurring or synthesized unsaturated esters with perfluoroaliphatic groups. The method comprises reacting, under free radical conditions, the unsaturated ester with the fluoroaliphaticsulfonyl halide compound. It is believed that halide radicals and fluoroaliphatic radicals derived from the fluoroaliphaticsulfonyl halide compound add across the one or more non-terminal carbon-carbon double bonds in the unsaturated ester to form fluoroaliphatic-group-substituted ester. The resulting esters are sometimes referred to herein, for brevity, as the first class of fluoroaliphatic-group-substituted esters of this invention.

Optionally, the method further comprises dehydrohalogenation of said first class of fluoroaliphatic-group-substituted esters to form a second class of fluoroaliphatic-group-substituted esters. Said second class of compounds is both substantially ethylenically unsaturated and substantially fluoroaliphatic-group-substituted at the sites of the fluoroaliphaticsulfonyl halide compound addition.

Optionally, the method further comprises hydrogenation of said second class of compounds to form a third class of fluoroaliphatic-group-substituted esters. Said third class of compounds is substantially saturated and fluoroaliphatic-group-substituted at the sites of the fluoroaliphaticsulfonyl halide compound addition. Alternatively, said third class of compounds can be prepared directly from said first class of compounds by reductive dehalogenation.

Some of the fluoroaliphatic-group-substituted esters of this invention can be represented by the following general formula

$(R^1)^n(Y)_n$ I

where, in the above formula,

$R^1$ is a polyvalent aliphatic radical which is a residue of an aliphatic polyhydroxy compound having about 3 to 12 carbon atoms (e.g., glycerol, sorbitan and sucrose) or a residue of an ethoxylated derivative of such polyhydroxy compound;

n is the valence of $R^1$ and is 3 to 8, and preferably 3 to 6;

Y is selected from the group consisting of $(-OC(O)R^2)_a$, $(-OH)_b$ and $[-OP(O)-(OR^3)]_c$ wherein

the sum of a, b and c equals n,

a is at least 1,

b is a number from 0 to 7,

c is 0 or 1,

$R^2$ is an aliphatic radical having 2 to about 90 carbon atoms, and preferably 4 to about 45 carbon atoms,

$R^3$ is an aliphatic radical having 1 to about 12 carbon atoms, and preferably 3 to 6 carbon atoms,

$R^2$ and $R^3$ may be straight chain, branched, and if sufficiently large cyclic or combinations thereof, such as alkylcycloaliphatic radicals, $R^2$ and $R^3$ may contain heteroatoms such as oxygen and nitrogen and may be substituted with chlorine and/or bromine atoms, and

at least one $R^2$ group is substituted with at least one, preferably 1 to 6, and more preferably 1 to 3, fluoroaliphatic groups.

The fluoroaliphatic group, also occasionally depicted herein as $R_f$, is an aliphatic, fluorinated, stable, inert, non-polar, monovalent organic radical. It can be straight chain, branched, and if sufficiently large, cyclic or combinations thereof such as fluoroalkylcycloaliphatic radicals. The skeletal chain of the $R_f$ radical can include heteroatoms such as oxygen and/or trivalent nitrogen. Generally, $R_f$ will have about 1 to 20 carbon atoms, preferably 3 to 12, and most preferably 6 to 12. Preferably, said groups are perfluorinated, that is they are saturated with fluorine atoms and contain no hydrogen. Generally, the $R_f$ group has the structure $-C_nF_{2n+1}$ .

One class of the fluoroaliphatic-group-substituted esters of this invention are those of the first class some of which compounds can be represented by the general formula:

$[CH_3(CH_2)_m[R^5(CH_2)_r]_p(CH_2)_n]_qZ$ II

where, in the above formula,

m and n are 1 to 12;

p is 1 to 6;

r is 0 or 1;

q is 1 to 6;

$R^5$ can be independently selected from the group consisting of (a)$-CH=CH-$ or $-CH_2CH_2-$ and (b) $-CH(X)-CH(R_f)-$or $-CH(R_f)CH(X)-$, where X is Cl or Br and $R_f$ is a fluoroaliphatic group or radical;

Z is an aliphatic residue derived from an aliphatic polyhydroxy compound having about 3 to 12 carbon atoms (e.g., glycerol, sorbitan, sorbitol) or a residue derived from the ethoxylated derivative of such polyhydroxy compound, and preferably is a glycerol-derived residue selected from the group consisting of

(a) $-COOCH_2CH(OCO-)CH_2OCO-$,

(b) $-COOCH_2CH(OH)CH_2OCO-$, and

(c) $-COOCH(OCO-)CH_2OP(O)(OR^6)O^-\ (M^{+1/v})_y$,

where M is H or a metal or ammonium cation of valence v, e.g., sodium, potassium, magnesium or aluminum, y is 0 or 1 and $R^6$ is selected from the group consisting of H, $-CH_2CH(N^+H_3)C(O)O^-$, $-CH_2CH_2N^+(CH_3)_3$, $-CH_2CH_2N^+H_3$ and $-C_6H_6(OH)_5$ (i.e., inositol-derived radical).

The total number of carbon atoms in $CH_3(CH_2)_m[R^5(CH_2)_r]_p(CH_2)_n-$ can be 5 to about 90, and preferably is 15 to about 45.

Another class of the fluoroaliphatic-group-substituted esters of this invention are those of the second class some of which compounds can be represented by the general formula:

$[CH_3(CH_2)_m[R^7(CH_2)_r]_p(CH_2)_n]_qZ$ III

where, in the above formula,

m, n, p, r, q, and Z are as defined for formula II; and

$R^7$ is independently selected from the group consisting of (a)-CH = CH- or $-CH_2CH_2-$ and (b) $-CH = C(R_f)-$ or $-C(R_f) = CH-$, where $R_f$ is fluoroaliphatic group or radical.

The total number of carbon atoms in $CH_3(CH_2)_m[R^7(CH_2)_r]_p(CH_2)_n-$ can be 5 to about 90, and preferably is 12 to about 45.

Another class of the fluoroaliphatic-group-substituted esters of this invention are those of the third class some of which compounds can be represented by the general formula:

$[CH_3(CH_2)_m[R^8 (CH_2)_r]_p(CH_2)_n]_qZ$ IV

where, in the above formula,

m, n, p, r, q and Z are as defined for formula II; and

$R^8$ is independently selected from the group consisting of (a) -CH = CH- or $-CH_2CH_2-$ and (b) $-CH_2CH(R_f)-$or $-CH(R_f)CH_2-$, where $R_f$ is fluoroaliphatic group or radical.

The total number of carbon atoms in $CH_3(CH_2)_m[R^8(CH_2)_r]_p(CH_2)_n-$ can be 5 to about 90, and preferably is 15 to about 45.

The fluoroaliphatic-group-substituted esters of this invention must contain at least one and preferably will contain a plurality of $R_f$ groups. One skilled in the art will appreciate that the optimum number and size of $R_f$ groups in the fluoroaliphatic-group-substituted esters will depend upon the application in which the ester will be used and that said number and size generally will be determined empirically. For example, if the fluoroaliphatic-group-substituted ester is to function as an emulsifier between a highly fluorinated liquid, e.g. perfluorotributylamine, and another immiscible liquid, e.g. water, said fluoroaliphatic-group-substituted ester may require larger and/or more $R_f$ groups than would be required in a fluoroaliphatic-group-substituted ester used as a lubricant between two solid surfaces. Typically said substituted esters will contain about 1 to 10 $R_f$ groups, and preferably 2 to 6 groups. Typically said substituted esters will contain about 6.0 to 57 weight percent fluorine and preferably about 54 weight percent fluorine.

Some particularly useful fluoroaliphatic-group-substituted esters are fluoroaliphatic-group-substituted monoacylglycerides, diacylglycerides, triacylglycerides, phosphoglycerides, sucrose monooleate, pentaerythritol monooleate, sucrose fatty acid esters (such as sucrose ricinoleate), sorbitan fatty acid esters (such as sorbitan monooleate), ethoxylated derivatives thereof and blends thereof.

Representative fluoroaliphatic-group-substituted esters include

Table 1

| No. | Structures |
|---|---|
| 1 | 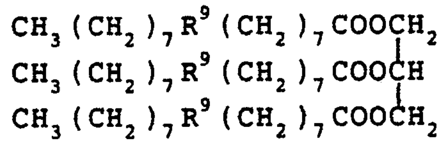 |
| 2 | 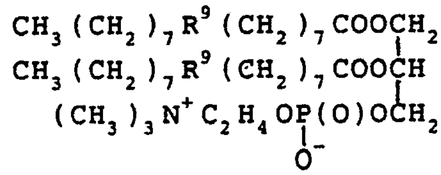 |
| 3 | 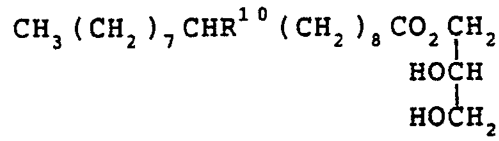 |
| 4 | 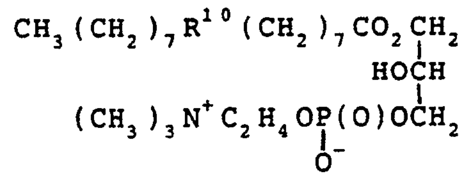 |
| 5 | 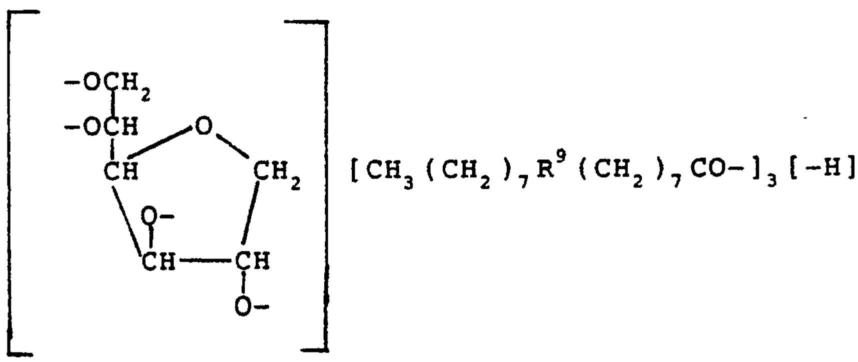 |
| 6 | 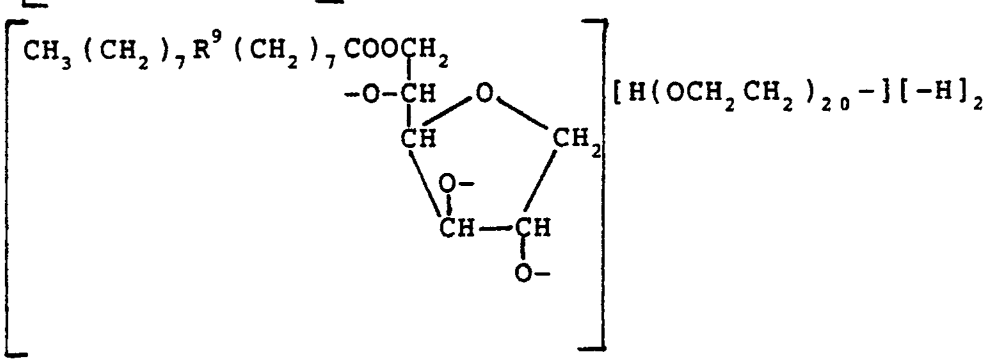 |

$R^9$ is $-CH(C_8F_{17})CH(Cl)-$ or $-CH(Cl)CH(C_8F_{17})-$
$R^{10}$ is $-CH(C_4F_9)CH(Br)-$ or $-CH(Br)CH(C_4F_9)-$

The ethylenically unsaturated esters useful for preparing the fluoroaliphatic-group-substituted esters can be naturally occurring compounds or may be synthesized from aliphatic polyols and one or more organic acids, such as fatty acids, using conventional methods. In any case, the resulting unsaturated ester must have at least one acyl group derived from an ethylenically unsaturated fatty acid having one or more non-terminal carbon-carbon double bonds.

Representative aliphatic polyol compounds which could be used to synthesize the unsaturated esters include glycerol, erythritol, pentaerythritol, trimethylolpropane, xylose, xylitol, mannitol, sorbitol, sorbitan, glucose, fructose, inositol, ether derivatives of such polyols (e.g., methyl glucoside, methyl mannoside, propyl glucoside and benzyl glucoside), and poly(oxyalkylene) or "ethoxylated" derivatives of such polyols (e.g., poly(oxythelene) derivatives of glycerol or sorbitan) and related materials described, for example, in Kirk-Othmer Encyclopedia of Chemical Technology, 3rd Ed., 1, John Wiley & Sons (1978), pp. 754-789.

Representative ethylenically unsaturated fatty acids useful for synthesizing the unsaturated esters include palmitoleic, arachidonic, oleic, arachidic, lignoceric, linoleic, α-eleostearic, linolenic, butyric, caproic, caprylic, capric, ricinoleic, gadoleic, erucic, behenic, cetoleic and (all cis) 4,7,10,13,16,19 docosahexanenoic acids.

Representative ethylenically saturated fatty acids useful for synthesizing the unsaturated esters include lauric, myristic, palmitic and stearic.

Some of the unsaturated esters useful in this invention can be represented by the following general formula

$(R^{11})^n(Y)_n$ V

where, in the above formula,

$R^{11}$ is a polyvalent aliphatic radical which is a residue of an aliphatic polyhydroxy compound having about 3 to 12 carbon atoms (e.g., glycerol, sorbitan, sorbitol and sucrose) or a residue of an ethoxylated derivative of such polyhydroxy compound;

n is the valence of $R^{11}$ and is 3 to 8, and preferably 3 to 6; and

Y is selected from the group consisting of $(-OC(O)R^{12})_a$, $(-OH)_b$ and $[-OP(O)-(OR^{13})]_c$ wherein

the sum of a, b and c equals n,

a is at least 1,

b is a number from 0 to 7,

c is 0 or 1,

$R^{12}$ is an aliphatic radical having 1 to about 22 carbon atoms, and preferably 4 to about 17 carbon atoms,

$R^{13}$ is an aliphatic radical having about 1 to 12 carbon atoms, and preferably about 3 to 6 carbon atoms,

$R^{12}$ and $R^{13}$ can be straight chain, branched, and if sufficiently large, cyclic radicals or combinations thereof, such as alkylcycloaliphatic radicals and may contain heteroatoms such as oxygen and nitrogen, and

at least one $R^{12}$ must have at least one, preferably 1 to 6, and more preferably 1 to 3, non-terminal carbon-carbon double bonds which are reactive with fluoroaliphaticsulfonyl halide compound.

Representative unsaturated esters useful in this invention include monoacylglycerides, diacylglycerides, triacylglycerides, glycosylacylglycerides (i.e., those acylglycerides which contain a sugar in glycosidic linkage with the unesterified 3-hydroxyl group of a diacylglyceride), alkyl ether acylglycerides, phosphoglycerides (e.g., phosphatidyl choline), sorbitan fatty acid esters (e.g., sorbitan monooleate), sucrose fatty acid esters and ethoxylated derivatives thereof (e.g., poly(oxyethylene) sorbitan monooleate), wherein all of these esters have at least one acyl group derived from an ethylenically unsaturated fatty acid having at least one non-terminal carbon-carbon double bond which is reactive with fluoroaliphaticsulfonyl halide compound.

Many of the unsaturated esters useful in this invention are commercially available, e.g., from American Lecithin Company as "ALCOLEC 662" (lecithin); from Capital City Products Company as "CAPMUL GMO" (glyceryl monooleate), as "CAPMUL O" (sorbitan monooleate) and "CAPMUL POE-O" (polyoxyethylene (20) sorbitan monooleate); from Hodag Chemical Corporation as "HODAG GMO" (glycerol monooleate); from Lonza Incorporated as "GLYCOSPERSE" TO-20 (polyoxyethylene sorbitan trioleate), "GLYCOMUL" SOC (sorbitan sesquioleate) and "GLYCOMUL" TO (sorbitan trioleate); from ICI America Incorporated as "AHCO DFO-100" (polyoxyethylene (5) sorbitan monooleate), "AHCO EO-102" (polyoxyethylene sorbitol polyoleate), "ATLAS G-1045A" (polyoxyethylene sorbitol polyoleate-laurate) and "ATLAS G-1087"

(polyoxyethylene (40) sorbitol hexaoleate); from Emery Chemicals Personal Care and Specialties Group as "EMEREST" 2419 (glycerol dioleate), "EMEREST "2421 (glycerol monooleate) and "EMEREST" 2423 (glycerol trioleate); from CasChem, Incorporated as "FLEXRICIN" 13 (glyceryl monoricinoleate); from Amerchol Corporation as "GLUCAMATE" DO (methyl glucoside dioleate) and "GLUCAMATE" DOE-120 (ethoxylated (120) methyl glucoside dioleate); and from R.I.T.A. Corporation as "GRILLOTEN ZT12, "GRILLOTEN ZT40" and "GRILLOTEN ZT50" (sucrose ricinoleates).

Many of the unsaturated esters useful in this invention are naturally occurring suoh as animal and vegetable fats and oils and lecithin (e.g., soybean and egg lecithin), or are derived therefrom, e.g., poly-(oxyethylene) derivatives. Some commonly available vegetable oils and animal fats useful in this invention are described in Applewhite, T.H., Kirk-Othmer Encyclopedia of Chemical Technology, 9, pp. 804-805 (1980). Some the naturally occurring unsaturated esters actually contain a variety of unsaturated esters, e.g., crude lecithin.

One class of naturally occurring, unsaturated esters particularly useful in this invention are triacylglycerides and glycosylacylglycerides that can be represented by the general formula:

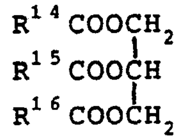

VI

where, in the above formula,

$R^{14}$ and $R^{15}$ can be independently selected from the group consisting of monovalent aliphatic radicals derived from saturated or unsaturated fatty acids such as palmitoleic, arachidonic, stearic, oleic, lauric, myristic, palmitic, arachidic, lignoceric, linoleic, $\alpha$-eleostearic, linolenic, butyric, caproic, caprylic, capric, ricinoleic, gadoleic, erucic, behenic, cetoleic, and (all cis) 4,7,10,13,16,19 docosahexanenoic, and

$R^{16}$ can be independently selected from the group consisting of aliphatic radicals of fatty acids, as described above, or monovalent radicals derived from monosaccharides and disaccharides. However, at least one of the groups, $R^{14}$, $R^{15}$, or $R^{16}$, must contain at least one non-terminal, carbon-carbon double bond which is reactive with fluoroaliphaticsulfonyl halide compound.

Another class of naturally occurring, unsaturated esters particularly useful in this invention are phosphoglycerides that be represented by the general formula:

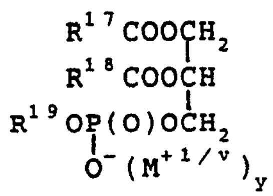

VII

where, in the above formula,

M is H or a metal or ammonium cation of valence v, e.g. sodium, potassium, magnesium or aluminum, and y is 0 or 1.

$R^{17}$ and $R^{18}$ are independently selected from the group consisting of monovalent, aliphatic radicals. Preferably, the monovalent aliphatic radicals are derived from fatty acids such as palmitic, stearic, oleic, linoleic and linolenic.

$R^{19}$ can be selected from a group consisting of H, $-CH_2CH(N^+H_3)C(O)O^-$, $(CH_3)_3N^+C_2H_4$-, $H_2N^+C_2H_4$- and $(HO)_5C_6H_6$-. However, either $R^{17}$ or $R^{18}$ must contain at least one non-terminal carbon-carbon double bond which is reactive with fluoroaliphaticsulfonyl halide compound.

One particularly useful naturally occurring unsaturated ester is soybean lecithin. Soybean lecithin comprises a blend of phosphoglycerides and fats or oils that can be precipitated from soybean oil by a degumming procedure. The degumming procedure generally yields product containing approximately 65 weight percent phospholipids and 35 weight percent raw soybean oil. Depending on the source of the soybeans and the method used for the extraction of the lecithin, soybean lecithin composition may vary, particularly with respect to the total concentration of phospholipids in the lecithin. However, such variation is acceptable in the practice of this invention. Typically lecithin comprises about 41 weight percent

phosphatidylcholine, 34 weight percent phosphatidylethanolamine, 19 weight percent phosphatidylinositol, and 9 weight percent phosphatidyl glycerol. (See Rydhag, L., and Wilton, I., "The Function of Phospholipids of Soybean Lecithin in Emulsions", JAOCS, pp. 830-837 (August 1981)).

The fluoroaliphaticsulfonyl halide compounds employed as the fluorochemical reagents for the introduction of fluoroaliphatic groups into the unsaturated esters are fluoroaliphaticsulfonyl chloride or bromide compounds. Such compounds can be obtained from fluoroaliphaticsulfonyl fluorides which are prepared from the electrochemical fluorination (ECF) of aliphaticsulfonyl chlorides or fluorides (see U.S. Patent No. 2,732,398 (Brice et al.)) followed by reduction of the fluoroaliphatic sulfonyl fluoride with aqueous sodium sulfite and halogenation (see U.S. Patent No. 3,420,877 (Pavlik)). While blends of fluoroaliphaticsulfonyl chlorides and bromides may be used in this invention, preferably either chlorides or bromides will be employed.

Representative fluoroaliphaticsulfonyl halide compounds which can be prepared by the above reactions and which can be used in this invention include:

$C_8F_{17}SO_2Cl$

$C_8F_{17}SO_2Br$

$C_4F_9SO_2Cl$

$C_4F_9SO_2Br$

$CF_3SO_2Cl$

$(CF_3)_2CF(CF_2)_6SO_2Cl$

$C_3F_7SO_2Br$

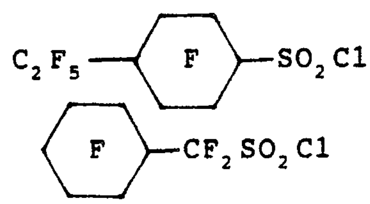

$C_{12}F_{25}SO_2Br$

$(C_2F_5)_2NCF_2CF_2SO_2Cl$

$C_3F_7OC_2F_4SO_2Cl$

The preferred method for preparation of the first class of fluoroaliphatic-group-substituted esters involves the reaction, under free radical conditions, of one or more fluoroaliphaticsulfonyl halide compounds with unsaturated ester. In this reaction, it is believed that chlorine or bromine radicals and fluoroaliphatic radicals add across one or more carbon-carbon double bonds in the unsaturated ester. Under certain circumstances, the addition of a fluoroaliphatic radical to a particular carbon atom of a carbon-carbon double bond may be favored over another, but generally, there is no regiospecificity. Preferably, the fluoroaliphaticsulfonyl halide compound is added to a solution containing the desired unsaturated ester and suitable free-radical-forming reagent. Suitable reaction solvents include ethyl acetate, octane, glacial acetic acid, acetonitrile, or blends thereof. Useful free-radical-forming reagents include organic peroxides such as benzoyl peroxide and di(tertiary butyl) peroxide, and organic azo compounds such as azobisisobutyronitrile (AIBN). Alternatively, ultraviolet radiation or electron beam radiation may be used to generate free radicals for this process.

The fluoroaliphaticsulfonyl halide compound can be employed in stoichiometric equality, deficiency, or excess (with respect to the number of carbon-carbon double bonds in the unsaturated ester) dependent upon the ease of reaction and upon the characteristics (e.g., the amount of fluoroaliphatic-group substitution) desired in the fluoroaliphatic-group-substituted ester. The reaction mixture is stirred at a suitable temperature, e.g., about 20 to 150 °C throughout the reaction period. Typically, if an organic peroxide such as benzoyl peroxide is used as a free-radical-forming reagent, the temperature of the reaction should be at or above the decomposition temperature of the peroxide, e.g., 60 to 120 °C. The reaction should be allowed to continue until the desired extent of reaction occurs. Ordinarily, this can be accomplished in about 1 to 4 hours. The product usually can be recovered by diluting the reaction mixture with a polar solvent (e.g., water), extracting the product with a suitable solvent (e.g., diethyl ether), and removing the remaining

solvent(s). Depending on the particular product, the desired degree of purity, and the available equipment, the product can be recovered from the remaining solvent(s) by evaporation, filtration, drying, or any combination thereof.

The fluoroaliphatic-group-substituted esters of the second class can be formed by reacting the desired fluoroaliphatic-group-substituted ester of the first class, dissolved in an anhydrous aprotic solvent (e.g., ethyl acetate), with a stoichiometric excess of an anhydrous aprotic base (e.g., N,N,N′,N′-tetramethyl ethylene diamine, 1,8-diazobicyclo [5,4,0]-undec-7-ene, triethylamine, or pyridine). This reaction typically proceeds at reflux temperature until completion. Generally the reaction goes to completion in 1 to 4 hours.

The third class of fluoroaliphatic-group-substituted esters of this invention are formed by catalytic hydrogenation of the fluoroaliphatic-group-substituted esters of the second class. In this method, a stoichiometric excess of hydrogen is reacted with the desired ester of the second class. Preferably, the second class ester is purified prior to hydrogenation, such as by passing it through a silica gel column. The purified compound can then be dissolved in a suitable solvent (e.g., absolute alcohol) and hydrogen pressured into the resulting solution. The hydrogenation reaction preferably takes place at ambient temperature and 40 psig (0.28 MPa).

Alternatively, the fluoroaliphatic-group- and bromine-substituted esters of the first class of this invention can be directly debrominated to form the fluoroaliphatic-group-substituted esters of the third class. In this method, the fluoroaliphatic-group- and bromine-substituted esters of the first class can be reacted with a stoichiometric excess of a debrominating agent such as a suitable metal hydride (e.g., tri n-butyltin hydride) at reflux temperature until the reaction is complete. Typically, the reaction goes to completion in 1 to 4 hours.

The compounds of this invention or blends of the compounds of this invention can be useful as dispersants or emulsifiers, lubricants, surfactants and anti-static agents. They are particularly useful as emulsifiers between fluorine-containing liquids and other non-miscible liquids. For example, fluoroaliphatic-group-substituted lecithin may be used as a dispersant in coating solutions used to apply the magnetic dispersions on magnetic recording media.

The objects and advantages of this invention are further illustrated in the following non-limiting examples. Unless otherwise indicated, all amounts, i.e., parts and percentages are by weight.

## EXAMPLE 1

This example illustrates the preparation of a perfluoroaliphatic-group-substituted monoacylglycerol.

17.4 g (0.05 mole) of glycerol monooleate was dissolved in 30 g of ethyl acetate. To this stirred solution, 23.0 g of perfluorohexanesulfonyl chloride (a 10% excess) was added and the solution was heated on a steam bath to reflux temperature. After the solution temperature reached 50 °C, a catalytic amount of azobisisobutyronitrile (AIBN), a free radical initiator, was added. After addition of the AIBN, a steady stream of $SO_2$ gas began to evolve from the reaction mixture.

After heating at reflux temperature for two hours, a small sample of the reaction mixture was withdrawn for gas chromatography (gc) analysis. Approximately a tenfold excess of a 25:75 boron trifluoride:methanol solution was added to the reaction mixture sample and the resulting mixture heated on a steam bath at its reflux temperature for 30 minutes to form the methyl ester derivatives. This mixture was mixed with water (25 °C), the ester product was extracted with hexane, and the hexane solution analyzed by gc. Analysis indicated a 45% conversion of unsubstituted oleate radical to perfluorohexyl- and chloro- substituted glyceride product.

To increase the extent of reaction, an additional catalytic amount of AIBN was added to the initial solution and heating continued for an additional 6 hours. Analysis of a sample of the resulting reaction solution (derivatized as described above) indicated a 71% conversion to the perfluorohexyl- and chloro-substituted glyceride.

The pale amber reaction solution was concentrated under reduced pressure to yield 34 g of a mixture which is believed to be mainly perfluorohexyl- and chloro-substituted monoacylglycerols,

$$CH_3(CH_2)_7CH(Cl)CH(C_6F_{13})(CH_2)_7CO_2CH_2$$
$$HOCH$$
$$HOCH_2$$

and

$$CH_3(CH_2)_7CH(C_6F_{13})CH(Cl)(CH_2)_7CO_2CH_2$$
$$HOCH$$
$$HOCH_2$$

and the starting glycerol monoleate.

## EXAMPLES 2-3

Following the general procedure of Example 1, perfluorobutyl and trifluoromethyl derivatives of trilinolein (i.e., trilinoleyl glycerol) were prepared. The results of this synthesis are summarized below in Table 2.

Table 2

| | Examples | |
|---|---|---|
| | 2 | 3 |
| Reactants | | |
| Trilinolein | 2.5 | 2.0 |
| $C_4F_9SO_2Cl$ | 5.9 | |
| $CF_3SO_2Cl$ | | 1.4 |
| Solvent, mL | | |
| Ethyl acetate | 20 | 20 |
| Reaction conditions | | |
| Time, hours | 3 | 2 |
| Temperature, °C | 75 | 75 |
| Conversion, % | 98 | 46[a] |
| Yield, g | 5.1 | 2.9 |

[a]Product comprised 33% mono-substituted trilinolein derivative and 17% di-substituted trilinolein derivatives.

## EXAMPLES 4-7

These examples illustrate the preparation of various fluoroaliphatic-group-substituted glycerides.

4.4 g (0.005 mole) of triolein (trioleoylglycerol), and 10 mL ethyl acetate were placed in a VYCOR silica flask. To this stirred solution, 2.5 g (0.005 mole) of perfluorooctanesulfonyl chloride was added. The reaction flask was placed about 5 cm from a General Electric "SUNLAMP", 275 watts, and heated on a steam bath. The reaction mixture was heated at reflux temperature for one hour. Analysis by gc of a small sample of the reaction mixture, as described in Example 1, indicated about a 75% conversion of triolein to a composition containing structure 1 shown in Table 2.

The reaction mixture was cooled to room temperature and diluted with water. From this mixture, the starting and product glycerides were extracted with diethyl ether, the extract dried over anhydrous sodium

sulfate, filtered and the remaining solvent removed under reduced pressure to yield 5.7 g of a mixture (1:3 weight ratio) of the starting triolein and its perfluorooctyl- and chloro- substituted adduct.

The adducts of various other glycerides were similarly prepared. The glycerides used, amounts of reactants, reaction conditions, percent conversion, yield, and adducts made are summarized in Table 3.

Table 3

| | Examples | | | |
|---|---|---|---|---|
| | 4 | 5 | 6 | 7 |
| Reactants, g | | | | |
| Triolein | 4.4 | | | |
| Sorbitan trioleate[a] | | 9.7 | | |
| Polyoxyethylene sorbitan monooleate[b] | | | 5.0 | |
| Dioleyl phosphatidyl choline | | | | 0.9 |
| $C_8F_{17}SO_2Cl$ | 2.5 | 17.3 | 5.0 | 1.4 |
| Solvent, mL | | | | |
| n-Octane | | 50 | | |
| Glacial acetic acid | | | | 10 |
| Acetonitrile | | | 30 | 20 |
| Ethyl acetate | 10 | | | |
| Reaction conditions | | | | |
| Time, hours | 1 | 1 | 1 | 1 |
| Temperature, °C | reflux | reflux | reflux | reflux |
| Conversion, % | 75 | 50 | 95 | 100 |
| Yield, g | 5.7 | c | 7 | c |
| Product no.[d] (Table 2) | 1 | 5 | 6 | 2 |

(a) Available commercially as SPAN 85
(b) Available commercially as TWEEN 80
(c) Yields not measured
(d) Has (or contains) structure indicated in Table 2

Example 8

In a VYCOR silica flask was combined 45 g of soybean lecithin and 45 mL of glacial acetic acid. To this stirred solution, heated to about 95°C, was added 69.7 g of perfluorooctanesulfonyl chloride and 1.2 g of di-(t-butyl)peroxide. An exothermic reaction took place with the temperature rising to 142°C. Heating (110-118°C) and stirring was continued for one hour. A small sample of the reaction mixture was analyzed using gc as described in Example 1. The analysis indicated that a 45 percent conversion of the glycerides to the perfluorooctyl-substituted adducts had occurred.

The reaction mixture was cooled to room temperature, mixed with cold tap water, the glycerides and adducts extracted with isooctane, dried over anhydrous sodium sulfate, filtered, and the remaining solvent removed under reduced pressure to yield a mixture (1:7.5 by weight) of the starting soybean lecithin and the adducts.

Example 9

Following the procedure of Example 8 a mixture of 645 g of soybean lecithin and 849 g perfluorooctanesulfonyl chloride, 1500 mL isooctane solvent, was heated for 7 hours at 95°C. Analysis of a sample of the reaction mixture by gc (using the procedure described in Example 1) showed a 77% conversion of the

starting soybean lecithin to the adducts. After extraction and drying as described in Example 1, 1230 g of a mixture of perfluorooctyl-substituted adducts and the starting soybean lecithin was produced.

## Example 10

Following the procedure of Example 8 a mixture of 10 g of soybean lecithin, 15.5 g of perfluorooctanesulfonyl chloride, and 25 mL of acetonitrile was heated for 2 hours at reflux temperature. Analysis by gc (using the procedure of Example 1) of a sample of the reaction mixture indicated that 70 percent of the soybean lecithin was converted to the adducts. After extraction and drying, 10.4 g of a mixture of perfluorooctyl-substituted adduct and starting soybean lecithin was produced.

## Examples 11-14

The following examples illustrate the preparation of fluoroaliphatic-group-substituted glycerides with fluoroaliphaticsulfonyl bromide compounds, and their subsequent dehydrobromination.

Fluoroaliphatic-group-substituted triolein was prepared by adding 11.0 g (0.03 mole) of perfluorobutanesulfonyl bromide to a solution of 8.85 g (0.01 mole) of triolein dissolved in 25 g isooctane. The reaction flask was wrapped with aluminum foil to exclude light. A slow evolution of $SO_2$ gas was observed along with a gradual darkening of the mixture overnight. Analysis of a sample showed about 50% conversion. A catalytic amount of benzoyl peroxide was added, and the resulting reaction mixture was heated in a steam bath at reflux temperature.

Analysis by gc of a sample of the reaction mixture indicated about a 90% conversion to the perfluorobutyl-substituted oleate. After extraction of the glycerides and drying, 16.5 g of a mixture of the product and starting triolein was produced.

The perfluorobutyl-substituted product was dissolved in ethyl acetate then reacted with various bases which function as dehydrobrominating agents under aprotic and anhydrous conditions. These reactions were allowed to proceed for an hour at reflux temperature. Analysis by gc indicated conversion to the following dehydrobrominated product

$$CH_3(CH_2)_7R^{20}(CH_2)_7COOCH_2$$
$$CH_3(CH_2)_7R^{20}(CH_2)_7COOCH_2$$
$$CH_3(CH_2)_7R^{20}(CH_2)_7COOCH_2$$

where $R^{20}$ is selected from $-CH(C_4F_9)CH(Br)-$, $CH(Br)CH(C_4F_9)-$, $-C(C_4F_9)=CH-$, $-CH=C(C_4F_9)-$ and $-CH=CH-$.

The amounts of fluoroaliphatic-group-substituted triolein, ethyl acetate, the type and amount of dehydrobrominating reagent, the reaction conditions, and percent conversion to unsaturated fluoroaliphatic-group-substituted product are shown in Table 4.

TABLE 4

| | Examples | | | |
|---|---|---|---|---|
| | 11 | 12 | 13 | 14 |
| Reactants, g | | | | |
| $C_4F_9$- and Br-substituted-triolein | 1.0 | 1.0 | 1.0 | 1.0 |
| Ethyl acetate | 4.0 | 4.0 | 4.0 | 4.0 |
| Dehydrobrominating reagent, g | | | | |
| N,N,N′,N′-tetramethylethylene diamine | 0.2 | | | |
| 1,8-diazobicyclo[5,4,0]-undec-7-ene | | 0.2 | | |
| Triethylamine | | | 0.2 | |
| Pyridine | | | | 0.2 |
| Reaction Conditions | | | | |
| Time, hours | 1 | 1 | 1 | 1 |
| Temperature | reflux | reflux | reflux | reflux |
| Conversion, % | 0 | 95 | 5 | 50 |

The data shows that 1,8-diazobicyclo[5,4,0]-undec-7-ene and pyridine were the most effective dehydrobrominating agents under these conditions.

EXAMPLE 15

This example illustrates the reductive debromination of fluoroaliphatic-group- and bromine-substituted glycerides of the first class to form fluoroaliphatic-group-substituted glycerides of the second class. To 1.78 g (0.001 mole) of the compound,

$$\begin{array}{l} CH_3(CH_2)_7R^{21}(CH_2)_7COOCH_2 \\ CH_3(CH_2)_7R^{21}(CH_2)_7COOCH \\ CH_3(CH_2)_7R^{21}(CH_2)_7COOCH_2 \end{array}$$

where $R^{21}$ is selected from $-CH(C_4F_9)CH(Br)-$, $-CH(Br)CH(C_4F_9)-$, and $-CH=CH-$, 20 g of tetrahydrofuran was added. To the resulting mixture, a 2 fold excess (2.62 g, 0.009 mole) of tri n-butyl tin hydride was added followed by 0.016 g of AIBN. This mixture was heated at reflux temperature for 4 hours. After four hours a small sample of the reaction mixture was withdrawn and analyzed by gc using a procedure similar to that described in Example 1. The analysis indicated the absence of bromine substituted fatty acids. Analysis by plasma chromatography and gc-mass spectroscopy further substantiated a loss of bromine and retention of the perfluorobutyl group.

EXAMPLE 16

The following Example describes the use of a fluoroaliphatic-group-substituted soybean lecithin to emulsify a perfluorochemical. A fluoroaliphatic-group-substituted lecithin similar to that prepared in Example 7 was mixed with a phosphate buffered saline solution (PBS). The PBS was prepared from 0.15M NaCl and 0.006 M $Na_2HPO_4$ and had a pH of about 7.0. About 3.5 mL of PBS was added for every 480 mg of fluoroaliphatic-group-substituted lecithin. The resulting mixture was sonicated using a microprobe in a Model 350 Bronson Sonifier operated at a setting of 5.

To the sonicated mixture, 2 mL of perfluorotripropyl amine was added. The mixture was kept cool in an ice bath while being sonicated for 4 cycles. Each cycle comprised 15 seconds of sonication followed by 1-2 minutes of resting. The resulting mixture was a very viscous dispersion that had the consistency of hand

lotion. No separation of the perfluorotripropyl amine was apparent indicating that the fluoroaliphatic-group-substituted lecithin was a useful emulsifier.

A similar emulsion made with unmodified soybean lecithin was also prepared according to the procedure described above, but this dispersion was less viscous than the emulsion made with fluoroaliphatic-group-substituted lecithin.

EXAMPLE 17

The following Example describes the catalytic hydrogenation of a glyceride of the second class to make a glyceride of the third class. An adduct of triolein and perfluorobutanesulfonyl bromide was made using a procedure similar to that described in Example 2. The reaction product was purified by passing it through a silica gel column (80 -20 mesh) using a chloroform-methanol eluting solvent mixture (90:10 to 80:20). The solvent was removed and the resulting adduct was mixed with anhydrous potassium acetate (0.08g), absolute alcohol (3A, 100 mL), a catalytic amount of palladium on carbon, and 2 drops of glacial acetic acid. The heterogeneous mixture was then hydrogenated by shaking with hydrogen gas at 40 psig (0.28 MPa) at ambient temperature for 2 hours. The resulting dark mixture was filtered through a diatomaceous-earth-coated filter pad and the solvent was removed at reduced pressure.

A small sample was analyzed by gc using the procedure described in Example 1. The analysis showed a shorter retention time of the resulting methyl esters when compared with esters of the starting bromo ester. Also, a loss of bromine and retention of the perfluorobutyl group was verified by plasma chromatographic analysis and gc-mass spectroscopy.

EXAMPLE 18

The following example illustrates the use of a fluoroaliphatic-group-substituted lecithin as a dispersant in a magnetic media dispersion.

Crude lecithin was prepared from a hydroxylated soybean oil available from Archer Daniels Midland Company as 3-900R-LECIN-H by extraction of the soybean oil. The resulting crude lecithin was washed with 10 g of acetone to remove soluble triglycerides and oil. The washed residue was mixed with 25 mL of glacial acetic acid, 15.5 g perfluorooctanesulfonyl chloride, and 0.2 g of di-t-butylperoxide. The mixture was then heated to 115-117 °C in an oil bath and held at that temperature for an hour. During the hour the evolution of $SO_2$ gas was observed.

A sample of the reaction mixture was analyzed by gc according to the procedure described in Example 1. The analysis indicated that about 60% of the fatty acids were substituted with perfluorooctane groups.

The reaction mixture was then quenched with water and the perfluorooctyl-substituted lecithin was isolated by extraction with ethyl acetate. The ethyl acetate solution was washed twice with an equal volume of water to remove the acetic acid. The ethyl acetate was then evaporated under reduced pressure and the resulting perfluorooctyl-substituted lecithin was dissolved in toluene to form a 30% by weight solution which was used to prepare the magnetic media dispersion.

A magnetic media dispersion was prepared by adding:

(a) 3.3 g of the 30% perfluorooctyl-substituted lecithin solution;

(b) 35.4 g of solvent (25% cyclohexanone, 25% toluene, 50% MEK),

(c) 25 g of $\delta$-$Fe_2O_3$ magnetic oxide pigment modified with cobalt,

(d) 7.26 of a 31.4 weight % solution of phenoxy resin (available from the Union Carbide Corporation as "UCAR" phenoxy resin PKHH) dissolved in MEK, and

(e) 29.2 g of a 17.9 weight % solution of urethane resin (available from B. F. Goodrich Company as "ESTANE" 5703) dissolved in an 80:20 toluene and MEK mixture

to a 114 mL stainless steel cylinder made by the Garner Division of the Pacific Scientific Company.

To the cylinder was added about 250 g of 3 mm diameter stainless steel spheres. The cylinder was closed and mounted on a "Red Devil" paint shaker where it was shaken for 40 minutes. After mixing, the mixture was strained through a wire mesh strainer to remove the stainless steel spheres and hand spreads of the dispersions were prepared.

To prepare a hand spread, about 50 grams of the dispersion was deposited on a polyethylene film about 15 cm wide. An identical piece of film was then laid over the top of the dispersion and the layered films were drawn under a fixed knife on a hand spreader apparatus. The gap under the edge of the spreader's fixed knife was set at 0.0076 cm. The resulting "hand spread" was allowed to air dry at ambient

temperature about 30 minutes before the it was cut into 0.64 cm wide strips. The magnetic properties of the strips were then measured using a B-H meter with a magnetic field setting of 3000 gauss.

Hand spreads of a 36 weight % solution of the unsubstituted crude lecithin described above were prepared according to the procedure described above and the magnetic properties of the strips of the handspread were measured as described above.

The viscosities of the dispersion containing the perfluorooctyl-substituted lecithin and the dispersion containing the unsubstituted lecithin were also measured using an ICI cone and plate viscometer set at a shear rate of 10,000 $sec^{-1}$.

The magnetic properties and viscosities of the dispersions are summarized below in Table 5.

TABLE 5

| | Perfluorooctane-substituted lecithin | Crude lecithin dispersion |
|---|---|---|
| viscosity ($sec^{-1}$) | 110 | 72 |
| Squareness[a] | 0.73 | 0.76 |
| $\Phi max$[b] | 0.414 | 0.416 |
| $\Phi_R$[c] | 0.302 | 0.324 |
| $1/G_n$[d] | 0.35 | 0.35 |
| $H_c$[e] | 339 | 338 |

(a) ($\Phi max/\Phi_R$) (i.e., the ratio of the maximum flux at saturation) ($\Phi max$) and the remanence flux ($\Phi_R$)
(b) the maximum flux at saturation
(c) remanence flux
(d) switching field distribution
(e) coercivity

The data indicates that the fluoroaliphatic-group-substituted lecithin is useful as a dispersant in a magnetic media solution.

The following trademarks are used in this application "ALCOLEC", "CAPMUL", "HODAG", "GLYCOSPERSE", "GLYCOMUL", "AHCO", "ATLAS", "EMEREST", "FLEXRICIN", "GLUCAMATE", "GRILLOTEN", "SPAN", "VYCOR", AND "ESTANE".

Various modifications and alterations of this invention will become apparent to those skilled in the art without departing from the scope and spirit of this invention.

## Claims

1. Fluorine-containing compounds comprising fluoroaliphatic-group-substituted esters derived from
(A) ethylenically unsaturated ester comprising a residue of an aliphatic polyol compound substituted with at least one acyl group derived from an ethylenically unsaturated fatty acid, said acyl group having at least one non-terminal carbon-carbon double bond, and
(B) fluoroaliphaticsulfonyl halide compound.

2. The compounds according to claim 1 wherein said ethylenically unsaturated ester is selected from the group consisting of monoacylglycerides, diacylglycerides, triacylglycerides, glycosylacylglycerides, alkyl ether acylglycerides, phosphoglycerides, sorbitan fatty acid esters, sucrose fatty acid esters and ethoxylated derivatives thereof, wherein said unsaturated ester has at least one acyl group derided from an ethylenically unsaturated fatty acid having at least one non-terminal carbon-carbon double bond.

3. The compounds according to claim 1 wherein said fluoroaliphaticsulfonyl halide compound is selected from the group consisting of compounds represented by the following formulas

$C_8F_{17}SO_2Cl$
$C_8F_{17}SO_2Br$
$C_4F_9SO_2Cl$
$C_4F_9SO_2Br$
$CF_3SO_2Cl$
$(CF_3)_2CF(CF_2)_6SO_2Cl$
$C_3F_7SO_2Br$

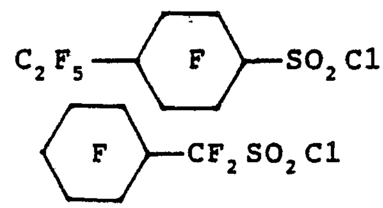

$C_{12}F_{25}SO_2Br$
$(C_2F_5)_2NCF_2CF_2SO_2Cl$
$C_3F_7OC_2F_4SO_2Cl$

4. The compounds according to claim 1 wherein said unsaturated ester is selected from the group consisting of triacylglycerides and glycosylacylglycerides which are represented by the following formula

$$\begin{array}{l} R^{14}COOCH_2 \\ R^{15}COOCH \\ R^{16}COOCH_2 \end{array}$$

where, in the above formula,
$R^{14}$ and $R^{15}$ are independently selected from the group consisting of monovalent aliphatic radicals derived from saturated or unsaturated fatty acids, and
$R^{16}$ is independently selected from the group consisting of aliphatic radicals derived from fatty acids and monovalent radicals derived from monosaccharides and disaccharides,
with the proviso that at least one of the groups, $R^{14}$, $R^{15}$ or $R^{16}$, contains at least one non-terminal, carbon-carbon double bond which is reactive with fluoroaliphaticsulfonyl halide compound.

5. The compounds according to claim 1 wherein said unsaturated ester is selected from the group consisting of phosphoglycerides represented by the following formula

$$\begin{array}{l} R^{17}COOCH_2 \\ R^{18}COOCH \\ R^{19}OP(O)OCH_2 \\ \quad O^-(M^{+1/v})_y \end{array}$$

where, in the above formula,
M is H or a metal or ammonium cation of valence v,
y is 0 or 1,

$R^{17}$ and $R^{18}$ are independently selected from the group consisting of monovalent, aliphatic radicals, and $R^{19}$ is selected from the group consisting of H, $-CH_2CH(N^+H_3)C(O)O^-$, $(CH_3)_3N^+C_2H_4-$, $H_2N^+C_2H_4-$ and $(HO)_5C_6H_6-$;

with the proviso that either $R^{17}$ or $R^{18}$ has at least one non-terminal carbon-carbon double bond which is reactive with fluoroaliphaticsulfonyl halide compound.

6. The compounds according to claim 1 wherein said ethylenically unsaturated ester is lecithin.

7. The compounds according to claim 1 represented by the general formula

$(R^1)^n(Y)_n$

where, in the above formula,

$R^1$ is a polyvalent aliphatic radical which is a residue of an aliphatic polyhydroxy compound having about 3 to 12 carbon atoms or a residue of an ethoxylated derivative of such polyhydroxy compound;

n is the valence of $R^1$ and is a number from 3 to 8; Y is selected from the group consisting of $(-OC(O)R^2)_a$, $(-OH)_b$ and $[-OP(O)-(OR^3)]_c$, where

the sum of a, b and c equals n,

a is at least 1,

b is a number from 0 to 7,

c is 0 or 1,

$R^2$ is an aliphatic radical having 2 to about 90 carbon atoms,

$R^3$ is an aliphatic radical having 1 to about 12 carbon atoms, and

at least one $R^2$ is substituted with at least one fluoroaliphatic group.

8. The compounds according to claim 1 selected from the group consisting of compounds represented by one of the following formulas

$[CH_3(CH_2)_m[R^5(CH_2)_r]_p(CH_2)_n]_qZ$

$[CH_3(CH_2)_m[R^7(CH_2)_r]_p(CH_2)_n]_qZ$

$[CH_3(CH_2)_m[R^8(CH_2)_r]_p(CH_2)_n]_qZ$

where, in the above formulas,

m and n are numbers from 1 to 12;

p is a number from 1 to 6;

r is 0 or 1,

q is a number from 1 to 6;

Z is an aliphatic residue derived from a polyhydroxy compound having about 3 to 12 carbon atoms or a residue derived from the ethoxylated derivative of such polyhydroxy compound;

$R^5$ is independently selected from the group consisting of (a) $-CH=CH$ or $-CH_2CH_2-$ and (b) $-CH(X)CH(R_f)-$ or $-CH(R_f)CH(X)-$, where X is Cl or Br and $R_f$ is a fluoroaliphatic radical;

$R^7$ is independently selected from the group consisting of (a) $-CH=CH-$ or $-CH_2CH_2-$ and (b) $-CH=C(R_f)-$or $-C(R_f)=CH-$, where $R_f$ is a fluoroaliphatic radical; and

$R^8$ is independently selected from the group consisting of (a) $-CH=CH-$ or $-CH_2CH_2-$ and (b) $-CH_2CH(R_f)-$or $-CH(R_f)CH_2-$, where $R_f$ is a fluoroaliphatic radical.

9. Emulsions comprising the compounds of claim 1.

10. Magnetic media dispersions comprising the compounds of claim 1.

Claims for the following Contracting State: ES

1. A method for preparing fluorine-containing compounds comprising fluoroaliphatic-group-substituted esters derived from

(A) ethylenically unsaturated ester comprising a residue of an aliphatic polyol compound substituted with at least one acyl group derived from an ethylenically unsaturated fatty acid, said acyl group having at least one non-terminal carbon-carbon double bond, and

(B) fluoroaliphaticsulfonyl halide compound,

by reacting under free-radical conditions the corresponding unsaturated ester and fluoroaliphaticsulfonyl halide compound, optionally dehydro-halogenating and further, optionally, hydrogenating the product of dehydro-halogenation or, optionally, reductively dehalogenating the initial product of the reaction under free-radical conditions.

2. A method according to claim 1 wherein said ethylenically unsaturated ester is selected from the group consisting of monoacylglycerides, diacylglycerides, triacylglycerides, glycosylacylglycerides, alkyl ether acylglycerides, phosphoglycerides, sorbitan fatty acid esters, sucrose fatty acid esters and ethoxylated derivatives thereof, wherein said unsaturated ester has at least one acyl group derived from an ethylenically unsaturated fatty acid having at least one non-terminal carbon-carbon double bond.

3. A method according to claim 1 wherein said fluoroaliphaticsulfonyl halide compound is selected from the

group consisting of compounds represented by the following formulas

$$C_8F_{17}SO_2Cl$$
$$C_8F_{17}SO_2Br$$
$$C_4F_9SO_2Cl$$
$$C_4F_9SO_2Br$$
$$CF_3SO_2Cl$$
$$(CF_3)_2CF(CF_2)_6SO_2Cl$$
$$C_3F_7SO_2Br$$

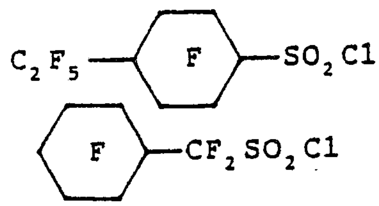

$$C_{12}F_{25}SO_2Br$$
$$(C_2F_5)_2NCF_2CF_2SO_2Cl$$
$$C_3F_7OC_2F_4SO_2Cl$$

4. A method according to claim 1 wherein said unsaturated ester is selected from the group consisting of triacylglycerides and glycosylacylglycerides which are represented by the following formula

$$\begin{array}{l} R^{14}COOCH_2 \\ \quad\quad\quad\;\; | \\ R^{15}COOCH \\ \quad\quad\quad\;\; | \\ R^{16}COOCH_2 \end{array}$$

where, in the above formula,
$R^{14}$ and $R^{15}$ are independently: selected from the group consisting of monovalent aliphatic radicals derived from saturated or unsaturated fatty acids, and
$R^{16}$ is independently selected from the group consisting of aliphatic radicals derived from fatty acids and monovalent radicals derived from monosaccharides and disaccharides;
with the proviso that at least one of the groups, $R^{14}$, $R^{15}$ or $R^{16}$, contains at least one non-terminal, carbon-carbon double bond which is reactive with fluoroaliphaticsulfonyl halide compound.

5. A method according to claim 1 wherein said unsaturated ester is selected from the group consisting of phosphoglycerides represented by the following formula

$$\begin{array}{r} R^{17}COOCH_2 \\ | \;\; \\ R^{18}COOCH \\ | \;\; \\ R^{19}OP(O)OCH_2 \\ | \quad\quad\quad\; \\ O^-(M^{+1/v})_y \end{array}$$

where, in the above formula,

M is H or a metal or ammonium cation of valence v,
y is 0 or 1,
$R^{17}$ and $R^{18}$ are independently selected from the group consisting of monovalent, aliphatic radicals, and
$R^{19}$ is selected from the group consisting of H, $-CH_2CH(N^+H_3)C(O)O^-$, $(CH_3)_3N^+C_2H_4$-, $H_2N^+C_2H_4$- and $(HO)_5C_6H_6$-;
with the proviso that either $R^{17}$ or $R^{18}$ has at least one non-terminal carbon-carbon double bond which is reactive with fluoroaliphaticsulfonyl halide compound.

6. A method according to claim 1 wherein said ethylenically unsaturated ester is lecithin.

7. A method according to claim 1 wherein the compounds produced are represented by the general formula
$(R^1)^n(Y)_n$
where, in the above formula,
$R^1$ is a polyvalent aliphatic radical which is a residue of an aliphatic polyhydroxy compound having about 3 to 12 carbon atoms or a residue of an ethoxylated derivative of such polyhydroxy compound;
n is the valence of $R^1$ and is a number from 3 to 8; Y is selected from the group consisting of $(-OC(O)R^2)_a$, $(-OH)_b$ and $[-OP(O)-(OR^3)]_c$, where
the sum of a, b and c equals n,
a is at least 1,
b is a number from 0 to 7,
c is 0 or 1,
$R^2$ is an aliphatic radical having 2 to about 90 carbon atoms,
$R^3$ is an aliphatic radical having 1 to about 12 carbon atoms, and
at least one $R^2$ is substituted with at least one fluoroaliphatic group.

8. A method according to claim 1 wherein the compounds produced are selected from the group consisting of compounds represented by one of the following formulas
$[CH_3(CH_2)_m[R^5(CH_2)_r]_p(CH_2)_n]_qZ$
$[CH_3(CH_2)_m[R^7(CH_2)_r]_p(CH_2)_n]_qZ$
$[CH_3(CH_2)_m[R^8(CH_2)_r]_p(CH_2)_n]_qZ$
where, in the above formulas,
m and n are numbers from 1 to 12;
p is a number from 1 to 6;
r is 0 or 1,
q is a number from 1 to 6;
Z is an aliphatic residue derived from a polyhydroxy compound having about 3 to 12 carbon atoms or a residue derived from the ethoxylated derivative of such polyhydroxy compound;
$R^5$ is independently selected from the group consisting of (a) -CH=CH- or $-CH_2CH_2$- and (b) $-CH(X)CH(R_f)$- or $-CH(R_f)CH(X)$-, where X is Cl or Br and $R_f$ is a fluoroaliphatic radical;
$R^7$ is independently selected from the group consisting of (a) -CH=CH- or $-CH_2CH_2$- and (b) $-CH=C(R_f)$-or $-C(R_f)=CH$-, where $R_f$ is a fluoroaliphatic radical; and
$R^8$ is independently selected from the group consisting of (a) -CH=CH- or $-CH_2CH_2$- and (b) $-CH_2CH(R_f)$-or $-CH(R_f)CH_2$-, where $R_f$ is a fluoroaliphatic radical.

9. Use in compositions as emulsifiers, surfactants, lubricants or anti-static agents of fluorine-containing compounds comprising fluoroaliphatic-group-substituted esters derived from
(A) ethylenically unsaturated ester comprising a residue of an aliphatic polyol compound substituted with at least one acyl group derived from an ethylenically unsaturated fatty acid, said acyl group having at least one non-terminal carbon-carbon double bond, and
(B) fluoroaliphaticsulfonyl halide compound.

10. Magnetic media dispersions containing a fluorine-containing compound which is a fluorine aliphatic-group-substitute ester derived from
(A) ethylenically unsaturated ester comprising a residue of an aliphatic polyol compound substituted with at least one acyl group derived from an ethylenically unsaturated fatty acid, said acyl group having at least one non-terminal carbon-carbon double bond, and
(B) fluoroaliphaticsulfonyl halide compound.